# EUROPÄISCHE PATENTSCHRIFT

(11) **EP 4 496 539 B1**
(45) Veröffentlichungstag und Bekanntmachung des Hinweises auf die Patenterteilung: **06.05.2026**
(21) Anmeldenummer: 23712289.0
(22) Anmeldetag: 21.03.2023
(51) Int. Cl.: A61F 5/01, F16F 9/34

(54) **VORRICHTUNG ZUR STABILISIERUNG VON KÖRPERGELENKEN UND/ODER ZUR UNTERSTÜTZUNG VON SPORTGERÄTEN**
DEVICE FOR STABILIZING BODY JOINTS AND/OR SUPPORTING SPORTS EQUIPMENT
DISPOSITIF POUR STABILISER LES ARTICULATIONS ET/OU SUPPORTER UN EQUIPEMENT SPORTIF

(30) Priorität: 21.03.2022 DE 102022106595
(43) Veröffentlichungstag der Anmeldung: 29.01.2025
(73) Patentinhaber: Betterguards Technology GmbH, 13585 Berlin (DE)
(72) Erfinder: BICHLER, Vinzenz, 10777 Berlin (DE); BUSCHINGER, Oscar, 10707 Berlin (DE); BORCHARDT, Elron, 10623 Berlin (DE)
(74) Vertreter: Okoampah, Rene
(86) Internationale Anmeldenummer: PCT/EP2023/057189
(87) Internationale Veröffentlichungsnummer: WO 2023/180306

(56) Entgegenhaltungen:
- WO-A1-2020/115227
- DE-A1- 102012 204 268
- DE-A1- 102018 131 463
- DE-B3- 102019 130 999
- DE-T5- 112018 003 791

## Beschreibung

### Technisches Gebiet

Die vorliegende Erfindung betrifft eine Vorrichtung zur Stabilisierung von Körpergelenken und/oder zur Unterstützung von Sportgeräten welche eine Aufnahme, die mit einem Füllmedium gefüllt ist, einen ersten Körper zum Interagieren mit dem Füllmedium, der in der Aufnahme verschiebbar angeordnet ist, einen Kraftübertragungskörper zum Übertragen einer äußeren Kraft auf den ersten Körper, und einen zweiten Körper zum Interagieren mit dem Füllmedium, welcher in der Aufnahme verschiebbar angeordnet ist, umfasst.

### Stand der Technik

Es ist bekannt Körpergelenke, Muskeln und Sehnen mittels Vorrichtungen, welche eine adaptive Bewegungsbegrenzung ermöglichen zu stabilisieren. Ferner ist es bekannt Sportgeräte, die bewegungsbedingten Belastungen ausgesetzt werden können, mit adaptiven Bewegungsbegrenzungsvorrichtungen zu versehen.

Unter anderem wird das adaptive Verhalten solcher Vorrichtungen dadurch erreicht, dass sich zwei Körper relativ zueinander bewegen, wobei sich zwischen den Körpern ein Füllmedium befindet. Dabei kann ein Körper der Vorrichtung eine Aufnahme bilden, die mit dem Füllmedium gefüllt ist. Der andere Körper kann einen Auszugskörper bilden, welcher in der Aufnahme bewegbar angeordnet ist. In dem Bereich zwischen der Aufnahme und dem Auszugskörper kann das Füllmedium strömen, wenn sich die beiden Körper relativ zueinander bewegen. Die Fließgeschwindigkeit des Füllmediums hängt entscheidend von der Querschnittfläche senkrecht zu einer relativen Verschieberichtung der Aufnahme und des Auszugskörpers ab. Diese zur Strömung für das Füllmedium bereitstehende Querschnittfläche wird auch als hydraulischer Durchmesser bezeichnet und ist letztendlich für das reaktive Verhalten der Vorrichtung bei äußerer Krafteinwirkung entscheidend. So kann durch die Wahl des hydraulischen Durchmessers der Widerstand festgelegt werden, den die Vorrichtung äußeren Kräften entgegenbringt. Die Vorrichtungen können zwischen zwei Körperstellen eines Anwenders oder zwei sich relativ zueinander bewegbaren Elementen eines Sportgeräts fixiert werden.

Werden über die beiden Körperstellen des Anwenders physiologische Kräfte, das heißt für das entsprechend zu stabilisierende Körperteil oder Bauteil unkritische Kräfte, in die Vorrichtung eingeleitet, so wird gemäß dem hydraulischen Durchmesser in der Vorrichtung eine entsprechende Relativbewegung der Aufnahme und des Auszugskörpers und damit eine Bewegung des zu stabilisierenden Körperteils zugelassen.

Werden hingegen unphysiologische Kräfte, das heißt für das entsprechend zu stabilisierende Körperteil oder Bauteil kritische Kräfte, in die Vorrichtung eingeleitet, ist aufgrund des hydraulischen Durchmessers eine Relativbewegung zwischen dem Auszugskörper und der Aufnahme nur noch unter sehr hohem Kraftaufwand möglich.

Aus EP 3 238 670 A1 und WO 2020/115227 A1 sind Vorrichtungen zur Stabilisierung von Körpeergelenken bekannt, die ein adaptives Verhalten in Abhängigkeit der Intensität einer einwirkenden Kraft ermöglichen.

Die Vorrichtungen umfassen jeweils eine Aufnahme, die mit einem Füllmedium gefüllt ist, einen ersten Körper zum Interagieren mit dem Füllmedium, wobei der erste Körper in der Aufnahme verschiebbar angeordnet ist, und ein Kraftübertragungskörper zum Übertragen einer äußeren Kraft auf den ersten Körper. Ein zweiter Körper zum Interagieren mit dem Füllmedium ist in der Aufnahme verschiebbar angeordnet, wobei der zweite Körper über ein Kopplungselement elastisch mit dem ersten Körper gekoppelt ist. Ferner weist der zweite Körper eine Durchlassöffnung auf, durch welche das Füllmedium strömen kann. Die Durchlassöffnung in dem zweiten Körper stellt einen hydraulischen Durchmesser für das Füllmedium bereit, durch welchen das Füllmedium fließen kann, solange zwischen dem ersten Körper und dem zweiten Körper ein Abstand besteht.

Der erste Körper bildet dabei einen Ventilkörper und der zweite Körper einen Ventilsitz, so dass ein Fluss des Mediums durch die Durchlassöffnung in Abhängigkeit von der Ventilstellung zugelassen oder unterbunden werden kann.

Auf den ersten Körper einwirkende äußere Kräfte können über das Kopplungselement auf den zweiten Körper übertragen werden. Entsprechend ist der erste Körper dazu in der Lage, den zweiten Körper mittels des Kopplungselements durch das Füllmedium zu schieben und/oder zu ziehen.

Dabei ist das Kopplungselement derart konfiguriert, dass es beim Einwirken einer äußeren Kraft auf den ersten Körper, im Bereich einer physiologischen Geschwindigkeit, eine Kraft auf den zweiten Körper überträgt, so dass dieser gemeinsam mit dem ersten Körper durch das Füllmedium bewegt werden kann.

Führt die über den ersten Körper und das Kopplungselement auf den zweiten Körper wirkende Kraft zu kritischen relativen Verschiebegeschwindigkeiten in der Vorrichtung, das heißt zu unphysiologischen Geschwindigkeiten, gibt das Kopplungselement nach, wodurch sich der erste Körper auf den zweiten Körper zubewegt. Dadurch verringert sich der hydraulische Durchmesser, bis das durch die beiden Körper gebildete Ventil geschlossen ist.

Wenn kein hydraulischer Durchmesser mehr zur Verfügung steht, durch den das Füllmedium fließen kann, können der erste Körper und der zweite Körper nicht weiter durch das Füllmedium bewegt werden. Die Vorrichtung blockiert.

Es hat sich gezeigt, dass das Schaltverhalten der vorstehend erläuterten Vorrichtungen zwischen einer offenen und einer geschlossenen Ventilstellung auch wesentlich von den auftretenden Reibungskräften zwischen dem zweiten Körper und der inneren Oberfläche der Aufnahme beeinflusst wird. Den auf den zweiten Körper indirekt über den ersten Körper und das Kopplungselement wirkenden Kräften wirkt die Reibungskraft zwischen dem zweiten Körper und der inneren Oberfläche der Aufnahme entgegen, wodurch das Schließverhalten des Ventils unmittelbar beeinflusst wird.

### Darstellung der Erfindung

Ausgehend von dem bekannten Stand der Technik ist es eine Aufgabe der vorliegenden Erfindung, eine verbesserte Vorrichtung zur Stabilisierung von Körpergelenken oder zur Unterstützung von Sportgeräten bereitzustellen.

Die Aufgabe wird durch eine Vorrichtung zur Stabilisierung von Körpergelenken oder zur Unterstützung von Sportgeräten mit den Merkmalen des Anspruchs 1 gelöst. Vorteilhafte Weiterbildungen ergeben sich aus den Unteransprüchen, der Beschreibung und den Figuren.

Entsprechend wird eine Vorrichtung zur Stabilisierung von Körpergelenken und/oder zur Unterstützung von Sportgeräten vorgeschlagen, umfassend: eine Aufnahme, wobei die Aufnahme mit einem Füllmedium gefüllt ist, einen ersten Körper zum Interagieren mit dem Füllmedium, wobei der erste Körper in der Aufnahme verschiebbar angeordnet ist, einen Kraftübertragungskörper zum Übertragen einer äußeren Kraft auf den ersten Körper, einen zweiten Körper zum Interagieren mit dem Füllmedium, welcher in der Aufnahme verschiebbar angeordnet ist, wobei der zweite Körper über ein Kopplungselement elastisch mit dem ersten Körper koppelbar ist, wobei zumindest der erste Körper mindestens eine Durchlassöffnung aufweist, durch welche das Füllmedium strömen kann. Erfindungsgemäß ist der zweite Körper innerhalb der Durchlassöffnung des ersten Körpers verschiebbar aufgenommen, wobei der erste Körper im Bereich der Durchlassöffnung einen Ventilsitz umfasst und der zweite Körper einen Ventilkörper bildet, so dass ein Fluss des Füllmediums durch die Durchlassöffnung in Abhängigkeit von der Ventilstellung zugelassen oder unterbunden werden kann.

Mittels der vorstehend beschriebenen Vorrichtung ist es möglich, das unmittelbare Schaltverhalten zwischen dem ersten Körper und dem zweiten Körper von dem Einfluss der Reibungskraft zwischen dem ersten Körper und der Aufnahme zu entkoppeln. Wird der erste Körper mittels des Kraftübertragungskörpers durch die Aufnahme bewegt, strömt das Füllmedium durch die Durchlassöffnung und umströmt dabei den zweiten Körper. In Abhängigkeit der Kräfte, die durch das umströmende Füllmedium auf den zweiten Körper wirken, kann dieser entgegen einer Spannkraft des Kopplungselements auf den Ventilsitz zubewegt werden, bis das Ventil vollständig geschlossen ist.

Somit hängt die Relativbewegung zwischen dem ersten Körper und dem zweiten Körper primär von der Spannkraft des Kopplungselements und der Geschwindigkeit des umströmenden Füllmediums bzw. der Strömungskraft des Füllmediums auf den zweiten Körper ab. Reibungskräfte, die zwischen dem ersten Körper und einer inneren Oberfläche der Aufnahme wirken, haben keinen unmittelbaren Einfluss auf die Relativbewegung zwischen dem ersten Körper und dem zweiten Körper. Insbesondere wirken sich mögliche Schwankungen, der zwischen dem ersten Körper und der inneren Oberfläche der Aufnahme auftretenden Reibungskräfte, nicht auf das Schalt- bzw. Schließverhalten des zweiten Körpers gegenüber dem Ventilsitz des ersten Körpers aus. Schwankungen können beispielsweise in Form von reibungsverursachenden Materialpaarungen, Haftreibung oder Reibung verursacht durch eine Krümmung der Aufnahme oder eine Biegung der Vorrichtung auftreten.

Unter dem Begriff _{"}Durchlassöffnung" ist ein Durchlass, zumindest durch den ersten Körper gemeint, der einen Passierweg bzw. Strömungsweg für das Füllmedium bereitstellt. Die Durchlassöffnung kann tunnelförmig, kanalförmig, oder in Form eines anderweitig gearteten Durchgangs ausgebildet sein.

Der Begriff _{"}Kraftübertragungskörper" umfasst vorliegend allgemein ein längliches, biegeschlaffes, optional elastisches Element, welches die Form einer einzelnen Faser, eines Faserstrangs, eines Drahts, einer Kordel, eines Seils, eines Textilgewebes mit begrenzter Breite und festen, beidseitigen Webkanten oder dergleichen aufweisen kann. Alternativ kann der Kraftübertragungskörper auch als zugsteifes und Schubeigenschaften aufweisendes Stabelement ausgebildet sein. Bevorzugt ist das Stabelement dabei einstückig mit dem ersten Körper ausgebildet.

In einer bevorzugten Ausführungsform weist die Durchlassöffnung mindestens einen sich in einer relativen Verschieberichtung des zweiten Körpers gegenüber dem ersten Körper erstreckenden Führungsabschnitt auf. Dadurch kann der zweite Körper in der relativen Verschieberichtung geführt werden. Der Führungsabschnitt definiert eine innere Oberfläche der Durchlassöffnung des ersten Körpers, entlang welcher der zweite Körper gegenüber dem ersten Körper zwischen einer Ausgangsposition und einer geschlossenen Position der Vorrichtung bewegt werden kann.

In einer weiter bevorzugten Ausführungsform weist die innere Oberfläche der Durchlassöffnung eine Führung auf. Dadurch kann der zweite Körper in Bezug auf den Querschnitt der Durchlassöffnung mittig gehalten werden. Auf diese Weise kann sichergestellt werden, dass zwischen der inneren Oberfläche der Durchlassöffnung und dem zweiten Körper ein vordefinierter Abstand bereitgestellt wird, durch welchen das Füllmedium strömen kann. Dieser vordefinierte Abstand bildet den hydraulischen Durchmesser der Vorrichtung.

In einer bevorzugten Weiterbildung begrenzt der Ventilsitz einen relativen Verschiebeweg des zweiten Körpers gegenüber dem ersten Körper in der relativen Verschieberichtung.

In einer bevorzugten Weiterbildung sind die relativen Abmessungen des zweiten Körpers gegenüber der Durchlassöffnung derart ausgebildet, dass in der offenen Ventilstellung ein Fluidfluss des Füllmediums im Bereich zwischen dem zweiten Körper und der inneren Oberfläche der Durchlassöffnung zugelassen werden kann. Dadurch kann der zweite Körper beim Einwirken einer äußeren Kraft auf die Vorrichtung mittels des Füllmediums umströmt werden. Die Umströmung des zweiten Körpers kann in Abhängigkeit der Größe bzw. der Geschwindigkeit, mit welcher eine äußere Kraft auf die Vorrichtung einwirkt, zu einer Strömungsgeschwindigkeit bzw. einer Strömungskraft des Füllmediums führen, die den zweiten Körper hinzu dem Ventilsitz des ersten Körpers bewegt.

In einer weiteren bevorzugten Ausführungsform wird der zweite Körper in einer Ausgangsposition der Vorrichtung vom Kopplungselement in einer offenen Ventilposition gehalten. Dadurch kann sichergestellt werden, dass in einer Ausgangsposition der Vorrichtung für das Füllmedium ein Strömungsweg durch die Durchlassöffnung des ersten Körpers bereitgestellt ist.

In einer bevorzugten Ausgestaltung ist das Kopplungselement dazu konfiguriert, dass beim Einwirken von physiologischen Kräften oder Geschwindigkeiten auf die Vorrichtung der zweite Körper durch das Kopplungselement entgegen einer Strömungsrichtung des Füllmediums in der offenen Ventilposition gehalten werden kann, und beim Einwirken von unphysiologischen Kräften oder Geschwindigkeiten auf die Vorrichtung die Strömungskraft des Füllmediums den zweiten Körper entgegen einer Spannkraft dem Kopplungselement in die geschlossene Ventilposition verschieben und darin halten kann.

In einer bevorzugten Weiterbildung ist der zweite Körper derart in der Durchlassöffnung des ersten Körpers aufgenommen, dass zumindest ein Bereich der Oberfläche des zweiten Körpers in einer offenen Ventilstellung mit Füllmedium umströmbar ist. Dadurch ist es möglich, dass durch die Umströmung des zweiten Körpers mit dem Füllmedium auf den zweiten Körper einwirkende Strömungskräfte auftreten können. Sofern die auf den zweiten Körper wirkenden Strömungskräfte größer einer Spannkraft des Kopplungselements sind, kann der zweite Körper in Richtung des Ventilsitzes des ersten Körpers bewegt werden, bis die Durchlassöffnung des ersten Körpers vollständig geschlossen ist.

In einer weiter bevorzugten Ausführungsform erstreckt sich die umströmbare Oberfläche des zweiten Körpers von einem ersten Oberflächenabschnitt, der mindestens einer ersten Endöffnung der Durchlassöffnung zugewandt ist, hinzu einem zweiten Oberflächenabschnitt, der mindestens einer zweiten Öffnung oder Endöffnung der Durchlassöffnung zugewandt ist. Eine Umströmung des zweiten Körpers von dessen ersten Oberflächenabschnitt hinzu dessen zweiten Oberflächenabschnitt, kann in Abhängigkeit der Strömungsgeschwindigkeit bzw. Anströmgeschwindigkeit des Füllmediums, der Beschaffenheit des Füllmediums und der Form des zweiten Körpers dazu führen, dass auf den ersten Oberflächenabschnitt, d. h. den Anströmabschnitt, eine größere Druckkraft wirkt als auf den zweiten Oberflächenabschnitt. Die aus der Druckdifferenz resultierende Kraft wirkt dabei in Richtung der Strömung des Füllmediums und ist bei einer Strömung in Richtung des Ventilsitzes für das Verschieben des zweiten Körpers hinzu dem Ventilsitz verantwortlich.

In einer bevorzugten Ausgestaltung umfasst die Vorrichtung einen ersten Körper mit einer Vielzahl an Ventilsitzen und einer der Anzahl der Ventilsitze entsprechenden Anzahl an zweiten Körpern. Die Ventilsitze können unterschiedlich große Durchlassöffnungen aufweisen und/oder in Reihe angeordnet sein. Die in Reihe angeordneten Ventilsitze sind in Abhängigkeit der Größe ihrer Durchlassöffnung stufenförmig angeordnet, sodass die Durchlassöffnungen der Ventilsitze von der ersten Endöffnung des ersten Körpers in Richtung der zweiten Endöffnung des ersten Körpers abnehmen. Analog sind die Oberflächen der zweiten Körper jeweils der Abmessung der entsperrenden Durchlassöffnung des entsprechenden Ventilsitzes angepasst. Dadurch kann ein stufenweises Schließen der Ventile ermöglicht werden.

In einer weiteren Ausführungsform weist der zweite Körper zumindest teilweise ein konvexes Anströmprofil oder ein konisches Anströmprofil auf. Dadurch kann der Effekt einer Druckdifferenz zwischen den an einer Anströmseite und an einer der Strömung abgewandten Seite des zweiten Körpers wirkenden Druckkräften begünstigt werden. Dabei bildet die der Anströmung abgewandte Seite die Seite des zweiten Körpers, welche hinzu dem Ventilsitz gewandt ist.

In einer bevorzugten Weiterbildung ist der zweite Körper kugelförmig.

Für eine bestmögliche Stabilisierung, ist es wichtig, den Zustand der geschlossenen Ventilstellung kontrolliert und gezielt auszulösen. Hierzu ist es vorteilhaft, den Einfluss der Reibung auf die Funktionalität des zweiten Körpers möglichst gering zu halten, sodass eine kugelförmige Ausformung des zweiten Körpers vorteilhaft ist. Entsprechend des Stokes'schen Reibungsgesetz ist die Reibung proportional zum Radius der Kugel, das einen weiteren Vorteil einer kompakten Bauweise begründet. Weiterhin hat die radialsymmetrische Ausformung einer Kugel den Vorteil, dass ein konstantes Strömungsprofil über die gesamte Oberfläche bereitgestellt ist, sodass das Schließen des Ventils unabhängig von einer möglichen Rotation des zweiten Körpers ist.

In einer weiter bevorzugten Ausführungsform umfasst der erste Körper einen Dichtabschnitt, bevorzugt einen adaptiven Dichtabschnitt, der zwischen dem ersten Körper und der inneren Oberfläche der Aufnahme umlaufend angeordnet ist, um den ersten Körper in der geschlossenen Ventilstellung gegenüber der Aufnahme abzudichten.

Durch den Dichtabschnitt kann sichergestellt werden, dass das Füllmedium bei einer offenen Ventilstellung durch die Durchlassöffnung des ersten Körpers geführt wird, um innerhalb der Aufnahme von einer Seite auf die andere Seite des ersten Körpers zu gelangen. Dabei kontaktiert der Dichtabschnitt die innere Oberfläche der Aufnahme und lässt eine Relativbewegung des ersten Körpers gegenüber der Aufnahme zu. Dieser Relativbewegung wird lediglich eine Reibungskraft entgegengesetzt, welche aus dem Kontakt des Dichtabschnitts und der inneren Oberfläche der Aufnahme resultiert.

Durch die Anordnung des Dichtabschnitts zwischen dem ersten Körper und der inneren Oberfläche der Aufnahme wirken hervorgerufenen Reibungskräfte nicht unmittelbar auf den zweiten Körper, welcher als Ventilkörper fungiert, ein. Somit wird das Schließverhalten zwischen dem zweiten Körper und dem Ventilsitz im Wesentlichen durch die aus der Umströmung des zweiten Körpers resultierenden Druckdifferenz beeinflusst. Insbesondere wirken sich mögliche Schwankungen der zwischen dem Dichtabschnitt und der inneren Oberfläche der Aufnahme auftretenden Reibungskräfte nicht auf das Schalt- bzw. Schließverhalten des zweiten Körpers gegenüber dem Ventilsitz des ersten Körpers aus. Der Dichtabschnitt kann auch als O-Ring ausgeformt sein.

In einer bevorzugten Weiterbildung umfasst der Dichtabschnitt ein Positionierteil, wobei die Durchlassöffnung zumindest teilweise durch das Positionierteil ausgebildet ist, und wobei der zweite Körper über ein Kopplungselement elastisch mit dem Positionierteil koppelbar ist.

Hierdurch kann das Positionierteil entsprechend zu dem ersten Körper ausgestaltet sein, sodass das Positionsteil anstelle des ersten Körpers den zweiten Körper über das Kopplungselement elastisch koppelt und mindestens eine Durchlassöffnung aufweist, damit das Füllmedium bei geöffneter Ventilstellung durch diese strömen kann. Weiterhin umfasst das Positionierteil im Bereich der Durchlassöffnung den Ventilsitz, so dass der zweite Körper, dessen Führungsabschnitt durch den ersten Körper bereitgestellt wird, den Fluss des Füllmediums in Abhängigkeit von der Ventilstellung zulassen oder unterbinden kann. Der erste Körper kann hierdurch einfacher und somit kostengünstiger ausgestaltet sein. Die Verbindung zwischen Positionierteil und erstem Körper wird bevorzugt formschlüssig beispielsweise mittels Schweißen, Schrauben oder Kleben bereitgestellt, wobei lediglich im Bereich des Kopplungselements geschweißt werden muss, sodass der Führungsabschnitt des zweiten Körpers durch die Verbindung nicht beeinflusst ist.

Weiterhin kann das Positionierteil so ausgeformt sein, dass der zweite Körper innerhalb der Durchlassöffnung des Positionierteils verschiebbar aufgenommen ist, wodurch eine vorteilhafte Führung des zweiten Körpers erreicht und geringe Fertigungstoleranzen des Positionierteils möglich sind. Die Verbindung des Positionierteils mit dem ersten Körper erfolgt bevorzugt formschlüssig, insbesondere durch Schweißen. Diese Ausgestaltung wird bevorzugt auch für die Unterstützung von Sportgeräten, beispielsweise Schuhen, insbesondere bei der Schnürung, den Schnürsenkeln und einer adaptiven Sohle, die beispielsweise eine geschwindigkeits- und/oder belastungsabhängige Biegesteifigkeit aufweist, verwendet.

In einer bevorzugten Ausführungsform weist der Kraftübertragungskörper oder der erste Körper einen Endanschlag auf, wobei der Kraftübertragungskörper von dem Endanschlag in Bewegungsrichtung bis zu einer Querschnittserweiterung mindestens eine Aussparung aufweist oder der erste Körper von dem Endanschlag in Bewegungsrichtung bis zu einer Querschnittserweiterung mindestens eine Aussparung aufweist und/oder der erste Körper vom Ventilsitz aus entlang der Bewegungsrichtung in Richtung des Kraftübertragungskörpers erstreckend mindestens eine Aussparung aufweist, sodass das Füllmedium durch die mindestens eine Aussparung strömen kann.

Diese Ausgestaltung ermöglicht eine besonders kompakte Ausformung des ersten Körpers und des Kraftübertragungskörpers. Hierdurch sind die Abstände zwischen der inneren Oberfläche der Aufnahme und ersten Körper und der inneren Oberfläche der Aufnahme und dem Kraftübertragungskörper ausreichend groß, sodass eine Biegung der Vorrichtung bzw. Krümmung der Aufnahme entlang der Bewegungsrichtung möglich ist. Somit kann die Vorrichtung dem Verlauf unterschiedlicher Formen besser folgen. Weiterhin formt die mindestens eine Aussparung mindestens eine Durchlassöffnung für das Füllmedium aus und ermöglicht eine form- oder kraftschlüssige Verbindung des die mindestens eine Aussparung aufweisenden Körpers mit einem die Durchlassöffnung begrenzenden Körper.

In einer weiter bevorzugten Ausführungsform umfasst der adaptive Abschnitt eine Dichtlippe, wobei die Dichtlippe bei geschlossener Ventilstellung mittels des Füllmediums gegen die innere Oberfläche der Aufnahme gedrückt werden kann. Ein auf die Vorrichtung einwirkender erhöhter Druck bei geschlossener Ventilstellung erhöht die Kraft, mit welcher die Dichtlippe gegen die innere Oberfläche der Aufnahme gedrückt wird.

In einer Weiterbildung unterteilt der erste Körper eine Kavität der Aufnahme in eine erste Kammer und eine zweite Kammer, wobei die Durchlassöffnung in einer offenen Ventilstellung einen Strom des Fluidmediums zwischen der ersten Kammer und der zweiten Kammer bereitstellen kann.

### Kurze Beschreibung der Figuren

Bevorzugte weitere Ausführungsformen der Erfindung werden durch die nachfolgende Beschreibung der Figuren näher erläutert. Dabei zeigen:
- Fig. 1A: schematisch eine perspektivische Ansicht einer Vorrichtung zur Stabilisierung von Körpergelenken und/oder Sportgeräten,
- Fig. 1B: schematisch eine seitliche Schnittansicht der Vorrichtung aus Fig. 1A in einem Ausgangszustand,
- Fig. 2A: schematisch eine Detailansicht der Vorrichtung gemäß Fig. 1B in einer Ausgangsposition,
- Fig. 2B: schematisch eine Detailansicht der Vorrichtung gemäß Fig. 1B in einer Blockierposition,
- Fig. 3: schematisch einen Teilausschnitt einer Schnittansicht einer modifizierten Vorrichtung,
- Fig. 4A: schematisch einen Teilausschnitt einer Schnittansicht einer alternativen Ausführung einer Vorrichtung,
- Fig. 4B: schematisch einen Teilausschnitt einer Schnittansicht einer alternativen Ausführung einer Vorrichtung,
- Fig. 5: schematisch eine Schnittansicht einer Vorrichtung ohne Dichtlippe,
- Fig. 6A: einen Teilausschnitt einer schematischen Schnittansicht einer alternativen Ausführung einer Vorrichtung mit Aussparungen im ersten Körper,
- Fig. 6B: schematisch Schnittansicht entlang einer Ebene senkrecht zur Bewegungsrichtung der in Fig. 6A gezeigten Vorrichtung,
- Fig. 6C: einen Teilausschnitt eines einstückig ausgeformten ersten Körpers und Kraftübertragungskörpers mit Anschlag,
- Fig. 6D: eine Schnittansicht eines Positionierteils, Kopplungselements und zweiten Körpers,
- Fig. 6E: einen Teilausschnitt einer schematischen Schnittansicht einer alternativen Ausführung einer Vorrichtung mit Aussparungen im ersten Körper, und
- Fig. 7: schematisch eine Schnittansicht eines Positionierteils in einer alternativen Ausführungsform.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen. Um Redundanzen zu vermeiden, wird auf eine wiederholte Beschreibung dieser Elemente teilweise verzichtet.

Die Funktion der vorliegenden Vorrichtung wird nachstehend anhand des Einsatzes im Sportbereich beschrieben, wobei auch ein Einsatz im Arbeitsbekleidungsbereich beispielsweise in Arbeitsstiefeln denkbar ist. Dabei kommt die Vorrichtung zur Dämpfung der Bewegung zweier Punkte eines Körpers, die sich relativ zueinander bewegen können, zum Einsatz. Ein solcher Körper kann beispielsweise ein Sportschuh sein, der gekoppelt mit der vorliegenden Vorrichtung einer Umknickbewegung entgegenwirken kann. Die vorliegende Vorrichtung ist jedoch nicht auf die hierin beschriebenen Einsatzgebiete beschränkt. So kann sie auch zwischen zwei Körperteilen eines Lebewesens angeordnet werden, um eine entsprechende Körperbewegung zu dämpfen. Alternativ kann die Vorrichtung auch in anderen Alltagsgegenständen verwendet werden, in welchen abrupt ansteigende Kräfte zwischen einem Körper oder einem Gegenstand gedämpft werden sollen. Weiterhin kann die Vorrichtung im Bereich Sportschuhe auch für die Schnürung, insbesondere die Schürsenkel und in Form einer adaptiven Sohle eingesetzt werden.

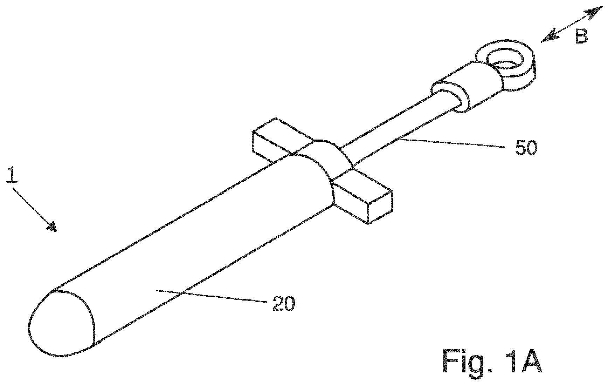

Fig. 1A ist eine perspektivische Ansicht einer Vorrichtung 1 zur Stabilisierung von Körpergelenken und/oder Sportgeräten zu entnehmen. Aus einer zylindrischen Aufnahme 20 ragt ein Kraftübertragungskörper 50 heraus. Dabei kann die Aufnahme 20 an einem Körperpunkt eines Anwenders oder Sportgeräts wie zum Beispiel eines Sportschuhs befestigt werden. Das Ende des Kraftübertragungskörpers 50, welches außerhalb der Vorrichtung 20 liegt, kann an einem zweiten Körperpunkt befestigt werden. Der erste Körperpunkt und der zweite Körperpunkt zeichnen sich dadurch aus, dass sie abrupten Relativbewegungen zueinander ausgesetzt sind. Die Richtung B stellt die Bewegungsrichtung der Vorrichtung dar. Alternative kann die Aufnahme auch quaderförmig ausgebildet sein.

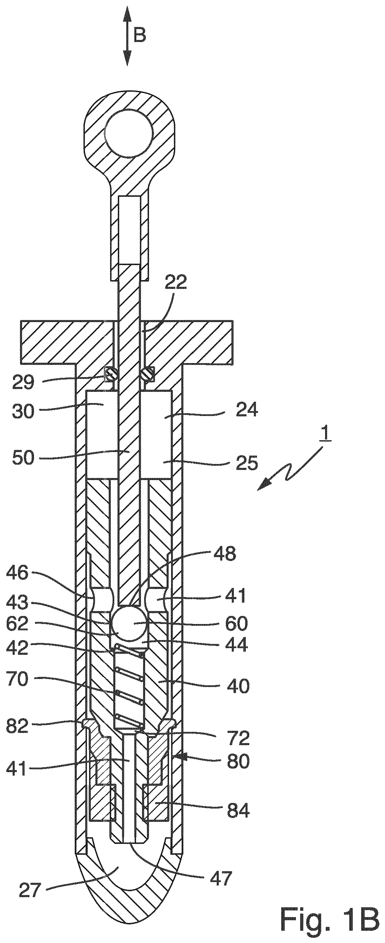

Fig. 1B zeigt eine Schnittansicht der Vorrichtung 1, welche sich in einem Ausgangszustand befindet. Die Aufnahme 20 weist eine Öffnung 22 auf, durch welche ein Kraftübertragungskörper 50 in den Innenraum 24 der Aufnahme 20 hineinragt. Bewegt sich der Körperpunkt, an welchem die Aufnahme 20 befestigt ist, relativ zu dem Körperpunkt, an dem das Kraftübertragungskörper 50 angeordnet ist, so bewegt sich das Kraftübertragungskörper 50 relativ zu der Aufnahme 20. Insbesondere kann sich das Kraftübertragungskörper 50 in einer Bewegungsrichtung B weiter in die Aufnahme 20 hinein oder weiter aus der Aufnahme 20 heraus bewegen.

Im Bereich der Öffnung 22 sind Dichteinsätze 29 angeordnet, welche den Innenraum 24 der Aufnahme 20 gegenüber der Umgebung abdichten, so dass das Füllmedium 30 im Innenraum 24 der Aufnahme 20 gehalten werden kann.

Die Aufnahme 20 der Vorrichtung 1 ist aus Edelstahl gefertigt. Alternativ kann die Aufnahme auch aus Kunststoff gefertigt sein. Unter anderem könne auch Faserverstärkte Kunststoffe zum Einsatz kommen. Alternativ kann die Aufnahme auch aus anderen Metallen wie zum Beispiel Aluminium oder Magnesium gefertigt sein. Darüber hinaus kann die Aufnahme auch aus Keramik gefertigt sein.

Das Kraftübertragungskörper 50 ist durch ein Kabel aus Draht gebildet und erstreckt sich von einem ersten Körper 40 durch eine erste Kammer 25 der Aufnahme 20 und schließlich durch die Öffnung 22 der Aufnahme 20. Alternativ kann das Kraftübertragungskörper 50 in Form eines Stabelements aus Kunststoff ausgebildet sein. Ferner kann das Kraftübertragungskörper 50 auch faserförmig ausgebildet sein, insbesondere aus Kunststofffasern, wie zum Beispiel Glasfaser, Kohlefaser, und dergleichen oder aus Naturfasern, wie zum Beispiel Hanffaser, Flachsfaser, und dergleichen. Des Weiteren kann das Kraftübertragungskörper 50 auch aus Metall, wie zum Beispiel Aluminium, Magnesium oder Edelstahl, gefertigt sein.

Ein Innenraum 24 der Vorrichtung 20 ist mit einem Füllmedium 30 gefüllt. Bei dem Füllmedium 30 handelt es sich um ein newtonsches Fluid wie zum Beispiel Silikonöl. Alternativ können auch dilatante Fluide als Füllmedium verwendet werden. Darüber hinaus kann auch ein scherverdickender Kunststoff eingesetzt werden. Dabei liegt der Kunststoff in Pulverform vor. Des Weiteren kann auch Sand als Füllmedium zum Einsatz kommen.

Darüber hinaus ist im Innenraum 24 der Vorrichtung 20 ein erster Körper 40 angeordnet, welcher in der Bewegungsrichtung B relativ zur Aufnahme 20 durch das Füllmedium 30 bewegbar ist. In der vorliegenden Ausführungsform ist der erste Körper 40 mit dem Kraftübertragungskörper 50 verpresst, so dass eine von dem Kraftübertragungskörper 50 ausgehende Kraft auf den ersten Körper 40 übertragen werden kann. Alternativ kann der erste Körper mit dem Kraftübertragungskörper 50 auch verschweißt, verklebt, einstückig ausgebildet oder anderweitig gekoppelt sein.

Der erste Körper 40 ist aus Kunststoff gefertigt. Alternativ kann der erste Körper auch aus einem Metall wie zum Beispiel Aluminium, Magnesium oder Stahl gefertigt sein.

Der Querschnitt des ersten Körpers 40 ist stets kleiner als der Querschnitt der Aufnahme 20, so dass das Füllmedium 30 zumindest teilweise auf der äußeren Umfangsoberfläche des ersten Körpers 40 relativ zur Aufnahme 20 strömen kann.

Ferner umfasst der erste Körper 40 eine Durchlassöffnung 41, durch welche das Füllmedium 30 strömen kann. Wird der erste Körper 40 entsprechend von einer, von dem Kraftübertragungskörper 50 ausgehenden Kraft relativ zur Aufnahme 20 bewegt, kann das Füllmedium 30 durch die Durchlassöffnung 41 des ersten Körpers 40 zwischen der ersten Kammer 25 und der zweiten Kammer 27 strömen. Entsprechend definiert die Durchlassöffnung 41 einen hydraulischen Durchmesser für das Füllmedium 30 durch den ersten Körper 40.

Nachfolgend wird die Fluidverbindung zwischen der ersten Kammer 25 und der zweiten Kammer 27 der Aufnahme 20 in eine Ausgangsposition der Vorrichtung 1 beschrieben. Wird das Kraftübertragungskörper 50 aus der Aufnahme 20 herausbewegt, kann das Füllmedium 30 auf dem Weg von der ersten Kammer 25 zur zweiten Kammer 27 zunächst durch den ersten hydraulischen Durchmesser zwischen der äußeren Umfangsoberfläche des ersten Körpers und der inneren Umfangsoberfläche der Aufnahme 20 strömen. Anschließend strömt das Füllmedium 30 durch eine seitliche Öffnung, d.h. eine erste Endöffnung 46, im ersten Körper 40 in die Durchlassöffnung 41. Schließlich kann das Füllmedium 30 aus einer zweiten Endöffnung 47 der Durchlassöffnung 41 in die zweite Kammer 27 der Aufnahme 20 strömen.

Des Weiteren ist in der Durchlassöffnung 41 des ersten Körpers 40 ein zweiter Körper 60 angeordnet, welcher relativ zum ersten Körper 40 in der Bewegungsrichtung B entlang eines Führungsabschnitts 43 bewegbar ist. Gemäß Figur 1B ist der zweite Körper 60 kugelförmig ausgebildet und weist einen Durchmesser auf, der geringfügig kleiner ist als der Durchmesser des inneren Umfangs einer Führungsoberfläche 44 der Durchlassöffnung 41. Der Abstand zwischen dem zweiten Körper 60 und der inneren Führungsoberfläche 44 bildet den tatsächlichen hydraulischen Durchmesser der Vorrichtung 1 durch welchen das Wirkmedium 30 hindurchströmen und somit den zweiten Körper 60 passieren kann.

Der zweite Körper kann alternativ auch andere Geometrien aufweisen wie zum Beispiel ein teilweise konvexes Profil oder ein konisches Profil und dergleichen. Der zweite Körper 60 ist aus Kunststoff gefertigt. Alternativ kann der zweite Körper auch aus einem Metall wie zum Beispiel Aluminium gefertigt sein.

Der Führungsabschnitt 43 der Durchlassöffnung 41 weist einen Endanschlag 48 für eine Ausgangsposition des zweiten Körpers 60 auf. Gemäß Figur 1B ist der Endanschlag 48 durch das Kraftübertragungskörper 50 gebildet, welches mit dem ersten Körper 40 pressgepasst ist. Dem Endanschlag 48 gegenüberliegend ist der Führungsabschnitt 43 durch eine Querschnittsverjüngung der Durchlassöffnung 41 begrenzt. Die Querschnittsverjüngung in der Durchlassöffnung 41 bildet einen Ventilsitz 42. Der zweite Körper 60 fungiert als Ventilkörper, welcher bei Kontakt mit dem Ventilsitz 42 die Durchlassöffnung 41 sperrt.

Darüber hinaus ist in der Durchlassöffnung 41 ein Kopplungselement 70 in Form einer Feder angeordnet, welches sich zwischen einem Federsitz 72 innerhalb des ersten Körpers 40 und dem zweiten Körper 60 erstreckt und den ersten Körper 40 mit dem zweiten Körper 60 koppelt. Das Kopplungselement 70 unterliegt einer Vorspannung, mittels welcher es den zweiten Körper 60 in der Ausgangsposition, d.h. gegen den Endanschlag 48 gedrückt, halten kann. Gemäß der vorliegenden Ausführungsform wird das Kopplungselement 70 in der Ausgangsposition aufgrund der Vorspannung des Kopplungselements 70 an dem zweiten Körper 60 gehalten. D.h. der zweite Körper 60 liegt lediglich an dem Kopplungselement 70 an.

Alternativ können das Kopplungselement 70 und der zweite Körper 60 auch dauerhaft miteinander verbunden oder gar einstückig ausgebildet sein. Das Kopplungselement 70 ist dann in der Lage den zweiten Körper 60 in einer Ausgangsposition zu halten, so dass in einer solchen Ausführungsform der zuvor beschriebene Endanschlag 48 nicht benötigt wird.

Gemäß der vorliegenden Ausführungsform ist das Kopplungselement 70 zusätzlich dazu konfiguriert, den zweiten Körper 60 in radialer Richtung der Durchlassöffnung 41 mittig zu halten. Dadurch kann sichergestellt werden, dass im Bereich des Führungsabschnitts 43 zwischen der Führungsoberfläche 44 und dem zweiten Körper 60 ein definierter Abstand bereitgestellt werden kann. Alternativ kann auf der Führungsoberfläche 44 auch eine Führung 45 vorgesehen sein, die den zweiten Körper 60 in Bezug auf die Längsachse der Durchlassöffnung 41 mittig hält.

Der erste Körper 40 weist auf seiner äußeren Umfangsoberfläche einen adaptiven Abschnitt 80 auf, der dazu eingerichtet ist, gegen die innere Umfangsoberfläche der Aufnahme 20 abzudichten. Der adaptive Abschnitt 80 ist dabei derart auf dem ersten Körper 40 positioniert, dass er zwischen den beiden Endöffnungen 46, 47 der Durchlassöffnung 41 angeordnet ist, wobei die erste Endöffnung 46 der ersten Kammer 25 und die zweite Endöffnung 47 der zweiten Kammer 27 fluidtechnisch zugeordnet ist. Somit unterbindet der adaptive Abschnitt 80 eine Fluidverbindung von der ersten Kammer 25 zur zweiten Kammer 27 auf der äußeren Umfangsoberfläche des ersten Körpers 40. Die einzige Fluid Verbindung zwischen der ersten Kammer 25 in der zweiten Kammer 27 ist durch die Durchlassöffnung 41 gegeben.

Bewegt sich der Kraftübertragungskörper 50 in Bewegungsrichtung B aus der Aufnahme 20 heraus, staut sich ein Teil des Wirkmediums 30 an dem adaptiven Abschnitt 80 auf und drückt diesen gegen die innere Umfangsoberfläche der Aufnahme 20. In der Ausführungsformen gemäß Figur 1B wird dieser Effekt durch eine Dichtlippe 82 am adaptiven Abschnitt 80 verstärkt.

Gemäß Figur 1B ist der adaptive Abschnitt 80 mittels eines Positionierteils 84 auf dem ersten Körper 40 gehalten das Positionierteils 84 ist in Form einer Schraubenmutter ausgebildet, welche auf ein Außengewinde an einem Endabschnitt des ersten Körpers 40 geschraubt ist. Alternativ kann der adaptive Abschnitt 80 auch mittels eines Sprengrings am ersten Körper 40 befestigt werden, wofür eine entsprechende Nut zur Aufnahme des Sprengrings im Endabschnitt des ersten Körpers 40 vorgesehen sein kann.

Die in den Fig. 1A und 1B gezeigte Vorrichtung 1 ist auf Zugbelastungen ausgelegt. D.h., auf Belastungen, welche aus einem Auseinanderbewegen des Körperpunkts, an dem die Aufnahme 20 befestigt ist und des Körperpunkts, an dem das Kraftübertragungskörper 50 befestigt ist, resultieren. Wird das Kraftübertragungskörper 50 aus der Vorrichtung 20 herausgezogen, so zieht es den ersten Körper 40 mit sich, wodurch es zu einem Fluss des Wirkmediums 30 von der ersten Kammer 25 entlang eines Teilabschnitts der äußeren Umfangsoberfläche des ersten Körpers 40, durch die Durchlassöffnung 41 an dem zweiten Körper 60 vorbei hin zu der zweiten Kammer 27 kommt.

Das Prinzip der in den Figuren 1A und 1B beschriebenen Vorrichtung 1 kann in gleicher Weise für eine auf Druckbelastung ausgelegte Vorrichtung zum Einsatz kommen. In diesem Fall ist das Kraftübertragungskörper 50 in From eines Druckstabs ausgebildet, welcher mit dem ersten Körper auf der Seite der zweiten Kammer verbunden ist. Entsprechend befindet sich auch der Austritt des Kraftübertragungskörpers 50 aus der Aufnahme im Vergleich zu den Fig. 1A und 1B auf der anderen Seite der Aufnahme.

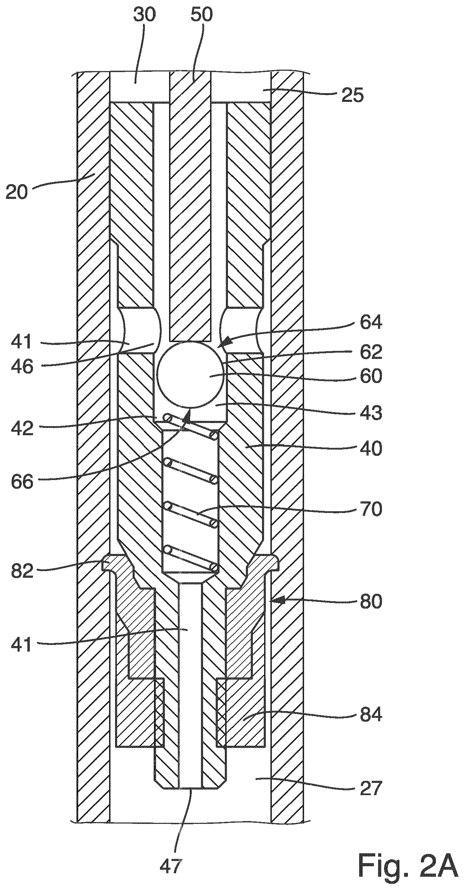

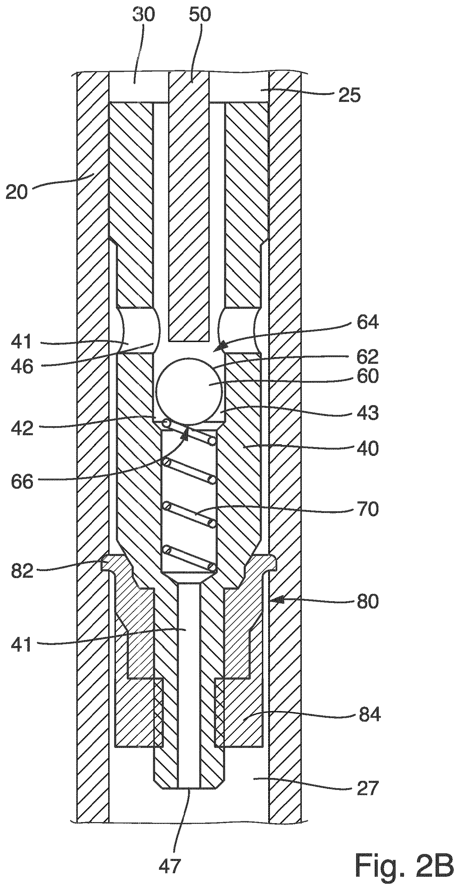

Fig. 2A und 2B zeigen Detailansichten der Vorrichtung 1 anhand welcher die Funktion der Vorrichtung 1 näher beschrieben wird. Wirkt eine Kraft im Bereich einer physiologischen Geschwindigkeit und wird das Kraftübertragungskörper 50 aus der Aufnahme 20 herausgezogen, strömt das Wirkmedium 30 von der ersten Kammer 25 entlang der äußeren Umfangsoberfläche des ersten Körpers 40, über die Endöffnung 46 in die Durchlassöffnung 41, vorbei an dem zweiten Körper 60 im Inneren der Durchlassöffnung 41, und schließlich über die Endöffnung 47 der Durchlassöffnung 41 in die zweite Kammer 27.

Die Oberfläche des zweiten Körpers 60 kann unterteilt werden in einen ersten Oberflächenabschnitt 64, der hin zu der ersten Kammer 25 orientiert ist, und in einen zweiten Oberflächenabschnitt 66, welcher hin zu der zweiten Kammer 27 orientiert ist. Eine Umströmung des zweiten Körpers 60 von dessen ersten Oberflächenabschnitt 64 hinzu dessen zweiten Oberflächenabschnitt 66, kann in Abhängigkeit der Strömungsgeschwindigkeit bzw. Anströmgeschwindigkeit des Füllmediums 30, der Beschaffenheit des Füllmediums 30 und der Form des zweiten Körpers dazu führen, dass auf den ersten Oberflächenabschnitt, d.h. den Anströmabschnitt, eine größere Druckkraft wirkt als auf den zweiten Oberflächenabschnitt. Die aus der Druckdifferenz resultierende Kraft wirkt dabei in Strömungsrichtung des Füllmediums 30 und kann bei einer Strömung in Richtung des Ventilsitzes 42 ein Verschieben des zweiten Körpers 60 hinzu dem Ventilsitz 42 bewirken.

Die Spannkraft des Kopplungselements 70 - hier die Federstärke eine Feder - ist derart gewählt, dass das Kopplungselements 70 erst beim Erreichen eines Schwellwerts, der auf den zweiten Körper 60 wirkenden Druckkraft, einfedert. Bei einer resultierenden Druckkraft unterhalb des Schwellwerts findet keine Relativbewegung des zweiten Körpers 60 gegenüber dem ersten Körper 40 statt. Bei einer Druckkraft oberhalb des Schwellwerts beginnt die Feder einzufedern, wodurch sich der zweite Körper 60 auf den Ventilsitz 42 des zweiten Körpers 40 zu bewegt, bis die Durchlassöffnung 41 vollständig geschlossen ist, wie in Figur 2B gezeigt. In diesem Zustand beträgt der hydraulische Durchmesser der Vorrichtung Null. Somit ist kein hydraulischer Durchmesser mehr vorhanden, durch den das Füllmedium 30 zwischen der ersten Kammer 25 und der zweiten Kammer 27 strömen kann.

Durch die Wahl von vergleichsweise geringen Federstärken bevorzugt 0.05 Nmm bis 1 Nmm lässt sich in Kombination mit der Vorspannung der Feder eine vergleichsweise schnelle Reaktionszeit der Vorrichtung von ungefähr 20ms, d.h. ein schnelles Schließen der Durchlassöffnung 41 realisieren. Ein Strömen des Füllmediums 30 von der ersten Kammer 25 in die zweite Kammer 27 ist nicht länger möglich, sodass der erste Körper 40 nicht länger relativ zur Aufnahme 20 bewegt werden kann. In diesem Zustand lässt die Vorrichtung 1 keine weitere Bewegung zwischen zwei zu unterstützenden/dämpfenden Körperpunkten zu.

Beispielsweise kann der Schwellwert der Druckkraft 4,5 N betragen und das System bei einem Sprung einer Widerstandskraft von 4,5 N auf 5 N sofort vollständig schließen.

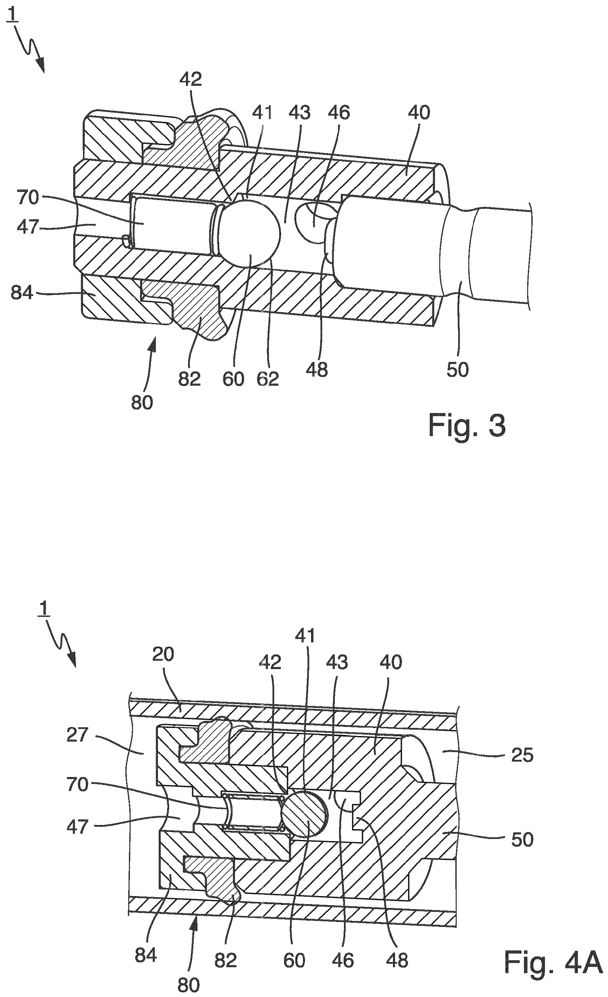

Figur 3 zeigt eine alternative Ausführungsform, die eine Modifikation der vorangegangen beschriebenen Vorrichtung 1 beschreibt.

Abweichend sind hierbei der erste Körper 40 und der Kraftübertragungskörper 50 mittels eines Spritzgussverfahrens aus Kunststoff hergestellt, wodurch kleinere Bauteilabmessungen unter Berücksichtigung der Fertigungstoleranzen möglich ist.

Im dargestellten Zustand ist der zweite Körper 60 auf den Ventilsitz 42 gedrückt, sprich die Durchlassöffnung 41 sperrt. Im nicht gezeigten Normalzustand kann das Kopplungselement 70 den zweiten Körper 60 gegen den Endanschlag 48 des Kraftübertragungskörpers 50 drücken.

Weiterhin ist es möglich im Führungsabschnitt 43 zusätzliche Führungspins anzuordnen. Diese dienen der Zentrierung des zweiten Körpers 60 und zum Ausgleich von Fertigungstoleranzen, insbesondere der Feder als Kopplungselement 70.

Nachfolgend ist in Figur 4A eine alternative Ausführungsform einer Vorrichtung 1 gezeigt.

Dabei ist ein Kraftübertragungskörper 50 und ein erster Körper 40 einstückig ausgeformt. Durch die Bauteilreduzierung wird eine platzsparende und kostengünstige Ausführung erreicht.

Weiterhin umfasst der erste Körper 40 einen adaptiven Dichtabschnitt 80, wobei die Durchlassöffnung 41 zumindest teilweise durch das Positionierteil 84 ausgebildet ist, sodass das Füllmedium 30 durch die Durchlassöffnung strömen kann. Der zweite Körper 60 ist über ein Kopplungselement 70 elastisch mit dem Positionierteil 84 koppelbar, dabei ist im vorliegenden Ausführungsbeispiel das Kopplungselement 70 eine Feder. Im Bereich der Durchlassöffnung 41 ist der Ventilsitz angeordnet, so dass der zweite Körper 60, der Ventilkörper, den Fluss des Füllmediums 30 durch die Durchlassöffnung 41 in Abhängigkeit von der Ventilstellung zulassen oder unterbinden kann.

Die einstückige Ausformung des Kraftübertragungskörpers 50 und des ersten Körpers 40 weist im Bereich des ersten Körpers 40 eine erste Endöffnung 46 auf. Die Durchlassöffnung 41 im ersten Körper 40 weist einen sich in einer relativen Verschieberichtung des zweiten Körpers 60 gegenüber dem ersten Körper 40 erstreckenden Führungsabschnitt 43 auf, um den zweiten Körper 60 in der relativen Verschieberichtung zu führen.

Vorteilhafterweise erfolgt bei dieser Ausgestaltung keine Schweißung im Bereich des Führungsabschnitts 43.

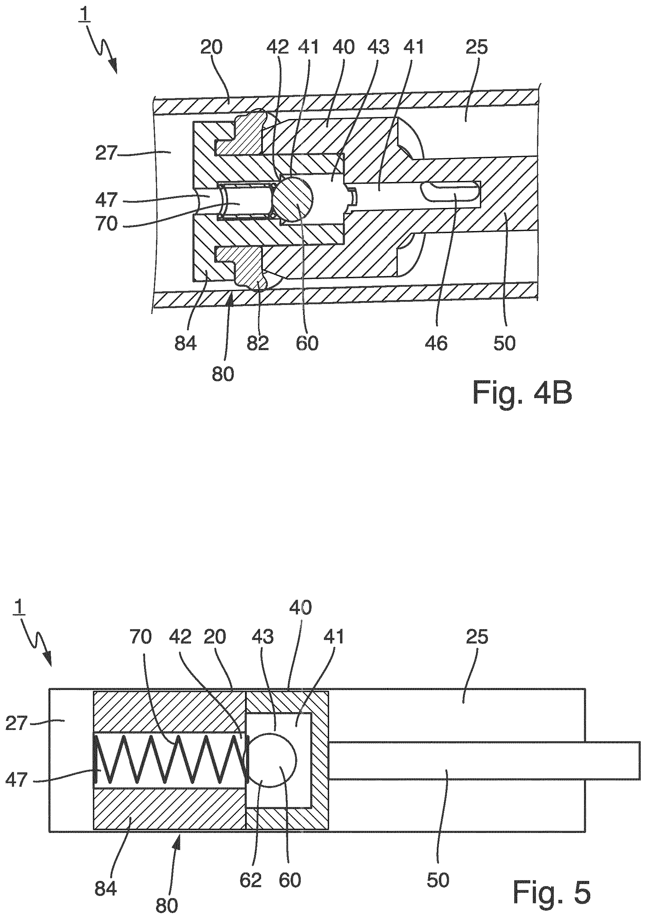

Figur 4B zeigt eine perspektivische Ansicht einer zur Figur 4A modifizierten Ausführungsform einer Vorrichtung 1 mit einem einstückig ausgeformten Kraftübertragungskörper 50 und ersten Körper 40.

Abweichend zur Ausführungsform in Figur 4A ist eine erste Endöffnung 46 im Bereich des Kraftübertragungskörpers 50 angeordnet, sodass die Durchlassöffnung 41 teilweise vom Kraftübertragungskörper 50 ausgebildet ist und ein Fluss des Füllmediums 30 in Abhängigkeit von der Ventilstellung zugelassen oder unterbunden werden kann.

Der zweite Körper 60 ist innerhalb des vom Positionierteil 84 ausgebildeten Bereich der Durchlassöffnung 41 verschiebbar aufgenommen. Die Durchlassöffnung 41 weist dazu einen sich in einer relativen Verschieberichtung des zweiten Körpers 60 gegenüber dem ersten Körper 40 erstreckenden Führungsabschnitt 43 auf, um den zweiten Körper 60 in der relativen Verschieberichtung zu führen.

Vorteilhafterweise bildet das Positionierteil 84 den Funktionsbereich des Kopplungselements 70 und den Führungsabschnitt 43 aus, sodass die Ausformung des Positionierteils 84 eine präzise Herstellung hinsichtlich geringer Fertigungstoleranzen und eine vorteilhafte Führung des zweiten Körpers 60 ermöglicht. Weiterhin führt die Ausformung dazu, dass keine Schweißung im Bereich des Kopplungselements notwendig ist,

In den Figuren 4A und 4B gezeigte Ausführungsformen sind neben der Stabilisierung von Körpergelenken auch vorteilhaft hinsichtlich der Unterstützung von Sportgeräten, insbesondere Schuhen, wobei die Ausführungen speziell bei Schnürsenkeln, der Schnürung und bei adaptiven Sohlen vorteilhaft sind.

Figur 5 zeigt eine weitere schematische Darstellung einer Vorrichtung 1.

Die Vorrichtung 1 umfasst die Aufnahme 20, einen Dichtabschnitt 80 umfassenden ersten Körper 40, einen Kraftübertragungskörper 50, wobei der erste Körper 40 und der Kraftübertragungskörper 50 auch einstückig ausgeformt sein können, ein nicht gezeigtes Kopplungselement 70 und den zweiten Körper 60.

Abweichend zu den vorherigen Ausführungsformen umfasst der Dichtabschnitt 80 keine Dichtlippe 82, sondern nur ein Positionierteil 84. Das Abdichten erfolgt beispielsweise mittels des Dichtabschnitts 80, insbesondere dem Positionierteil 84. Alternativ kann auch der erste Körper 40 so ausgeformt sein, dass dieser eine Dichtung zwischen einer ersten Kammer 25 und einer zweiten Kammer 27 bereitstellt, wobei auch eine kombinierte Abdichtung aus Dichtabschnitt 80 und ersten Körper 40 denkbar ist. Sollte der erste Körper 40 und der Kraftübertragungskörper 50 einstückig ausgeformt sein, ist die Abdichtung entsprechend darüber möglich. Als Folge dieser Dichtung würde sich ein Fluidfilm zwischen der inneren Oberfläche der Aufnahme 20 und dem zur Abdichtung ausgeformten Körper bilden, wobei der Fluidfilm die Funktionalität der Vorrichtung 1 nicht beeinflussen würde. Ohne eine Dichtlippe 82 kann eine deutlich vorteilhafte Dauerlaststabilität erreicht werden. Weiterhin wird die Anzahl benötigter Bauteile reduziert und die Fertigung kostengünstiger und einfacher.

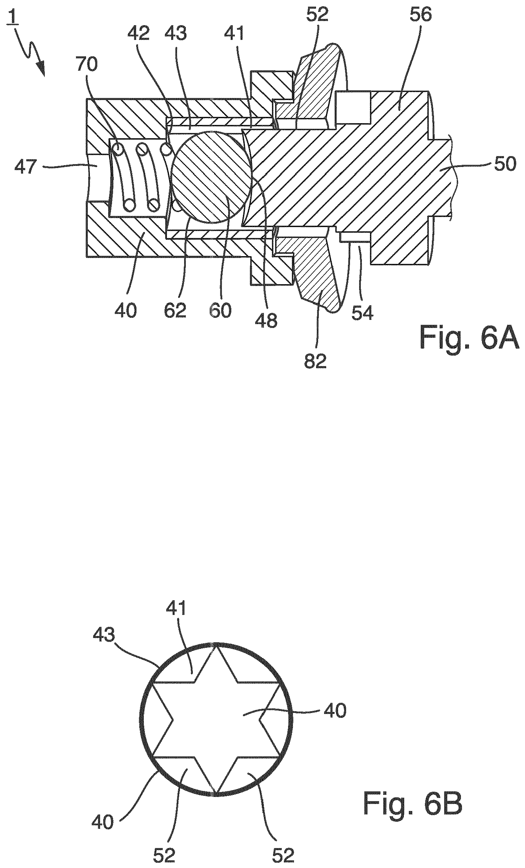

Figur 6A zeigt eine Schnittdarstellung durch eine alternative Ausführungsform einer Vorrichtung 1.

Die Vorrichtung 1 umfasst neben einer nicht gezeigten Aufnahme 20, einen zweiten Körper 60, ein Kraftübertragungskörper 50 und einen ersten Körper 40, wobei der erste Körper 40 einen adaptiven Dichtabschnitt 80 umfasst.

Der adaptive Dichtabschnitt 80 umfasst ein Positionierteil 84 und eine Dichtlippe 82, die vollumfänglich zwischen dem ersten Körper 40 und der inneren Oberfläche der Aufnahme 20 angeordnet ist und diese kontaktiert, wobei bei geschlossener Ventilstellung die Dichtlippe zusätzlich mittels des Füllmediums 30 gegen die innere Oberfläche der Aufnahme 20 gedrückt wird. Weiterhin weist der erste Körper 40 eine Querschnittserweiterung 56 auf, um die Dichtlippe 82 mittels des Positionierteils 84 axial zu positionieren. Die Anordnung der Dichtlippe ist nicht auf die hier gezeigte Ausführung beschränkt.

Weiterhin bildet der erste Körper mindestens eine Durchlassöffnung 41 aus, durch die das Füllmedium 30 strömen kann, wobei der erste Körper im Bereich der Durchlassöffnung 41 einen Ventilsitz 42 umfasst und der zweite Körper 60 innerhalb der Durchlassöffnung 41 des Positionierteils 84 verschiebbar aufgenommen ist. Die Durchlassöffnung 41 weist einen sich in einer relativen Verschieberichtung des zweiten Körpers 60 gegenüber dem ersten Körper 40 erstreckenden Führungsabschnitt 43 auf, um den zweiten Körper 60 in der relativen Verschieberichtung zu führen.

Der Kraftübertragungskörper 50 weist einen Endanschlag 48 auf, wobei der Kraftübertragungskörper 50 von dem Endanschlag 48 in Bewegungsrichtung B bis zu der Querschnittserweiterung 56 mindestens eine Aussparung 52, im vorliegenden Ausführungsbeispiel eine Mehrzahl von Aussparungen 52, aufweist, sodass das Füllmedium 30 durch die mindestens eine Aussparung 52 in Richtung des zweiten Körpers strömen kann. Die mindestens eine Aussparung 52 kann funktional als erste Endöffnungen betrachtet werden. Weiterhin weist der Kraftübertragungskörper im Bereich der Querschnittserweiterung 56 in Richtung des ersten Körpers eine Mehrzahl von Nuten 54 auf.

In der vorliegenden Ausführungsform bilden die Aussparungen 52 im Querschnitt betrachtet eine Sternform, wie in Figur 6B gezeigt, wobei die Erfindung nicht auf die in dieser Ausführungsform beschriebenen Form oder Anzahl an Aussparungen 52 beschränkt ist. Weiterhin ist es möglich die Aussparungen 52 in axialer Richtung in die Querschnittserweiterung 56 hinein zu erstrecken, sodass die Aussparungen 52 die Nuten 54 im Bereich der Querschnittserweiterung 56 ersetzen.

Vorteilhafterweise sind die Aussparungen 52 rotationssymmetrisch und äquidistant auf dem Umfang verteilt, sodass sich eine gleichmäßige Anströmung des zweiten Körpers 60 ergibt.

Dabei ist ein dem ersten Körper 40 zugewandte Teil des Kraftübertragungskörpers 50 teilweise mit dem ersten Körper 40 über den Führungsabschnitt 43 verbunden. Die Verbindung kann stoffschlüssig mittels Schweißen, beispielsweise Ultraschall- oder Laserschweißen oder als kraftschlüssige Verbindung mittels Pressen bereitgestellt werden, wobei der Kraftübertragungskörper 50 den Führungsabschnitt 43 nicht vollumfänglich kontaktiert.

Diese Ausgestaltung ermöglich eine sehr kompakte Ausformung des ersten Körpers 40 und des Kraftübertragungskörpers 50, sodass die Abstände zwischen der inneren Oberfläche der Aufnahme 20 und dem ersten Körper 40 bzw. dem Kraftübertragungskörper 50 eine Biegung der Vorrichtung 1 um die Verbindung im Bereich des Führungsabschnitts 43 entlang der Bewegungsrichtung B ermöglichen.

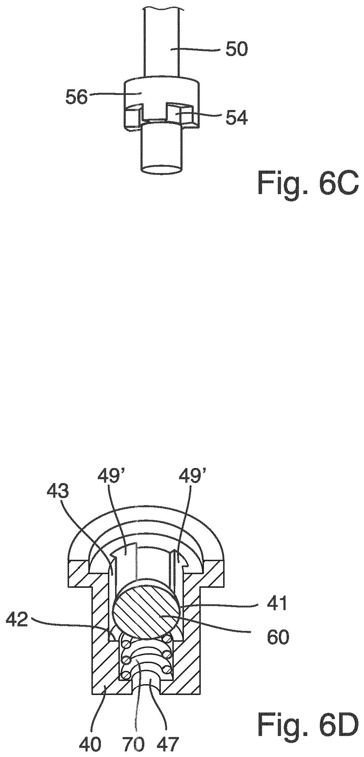

Figur 6C zeigt eine alternative Ausformung einer erfindungsgemäßen Vorrichtung 1.

Dabei ist der erste Körper 40, im Bereich vom Endanschlag 48 bis zu einer Querschnittserweiterung 56 in Bewegungsrichtung B erstreckend als Zylinder ausgeformt. Zusätzlich weist die Querschnittserweiterung 56 in Richtung des Endanschlags 48 eine Mehrzahl von Nuten 54 auf.

Wie in Figur 6D zu erkennen, ist mindestens eine Aussparung 49, im vorliegenden Ausführungsbeispiel eine Mehrzahl von Aussparungen 49 in einem Führungsabschnitt 43 angeordnet und bilden die Durchlassöffnungen 41, wobei analog zur Ausführungsform in Figur 6A und 6B der Kraftübertragungskörper 50 mit dem Führungsabschnitt 43 kraft- oder stoffschlüssig verbunden ist.

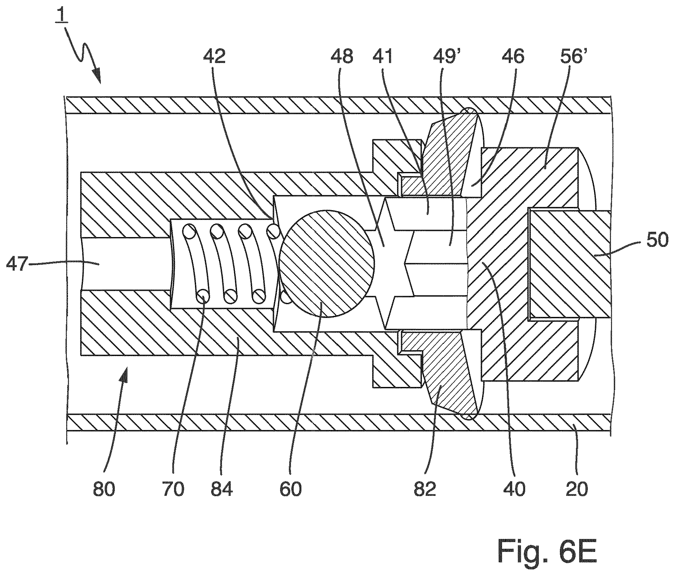

Figur 6E zeigt einen Teilausschnitt einer schematischen Schnittansicht einer alternativen Ausführung einer Vorrichtung 1 mit Aussparungen 49' im ersten Körper 40. Die Vorrichtung 1 umfasst einen in einer Aufnahme 20 verschiebbar angeordneten ersten Körper 40 und einen am ersten Körper 40 in Richtung der Öffnung 22 der Aufnahme 20 angeordneten Kraftübertragungskörper 50. Weiterhin umfasst der erste Körper 40 einen adaptiven Dichtabschnitt 80 umfassend ein Positionierteil 84 und eine Dichtlippe 82. Im Positionierteil 84 ist ein zweiter Körper 60 verschiebbar aufgenommen und über ein Kopplungselement 70 elastisch mit dem Positionierteil 84 koppelbar.

Der Kraftübertragungskörper 50 ist mit dem ersten Körper 40 verbunden, wobei die Verbindung formschlüssig beispielsweise mittels Schweißen oder kraftschlüssig beispielsweise mittels Pressen bereitgestellt werden kann.

Der erste Körper 40 weist von einem Endanschlag 48 in Bewegungsrichtung B bis zu einer Querschnittserweiterung 56' mindestens eine Aussparung 49' auf, sodass das Füllmedium durch die mindestens eine Aussparung strömen kann. In der gezeigten Ausführungsform bilden die mindestens eine Aussparung 49' mindestens eine Durchlassöffnung 41. Dazu ist der Kraftübertragungskörper 50 mit dem adaptiven Dichtabschnitt 80 verbunden, sodass der zweite Körper 60 mittels des Koppelungselements 70 gegen den Endanschlag 48 des ersten Körpers 40 in eine Ausgangsposition gehalten werden kann. Die Verbindung zwischen ersten Körper 40 und adaptiven Dichtabschnitt 80 kann formschlüssig beispielsweise mittels Schweißen oder kraftschlüssig beispielsweise mittels Pressen erfolgen.

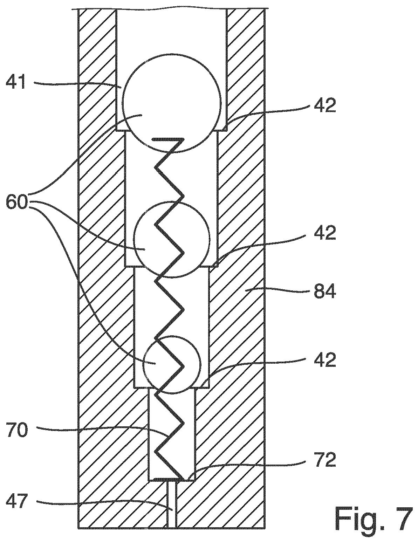

In Figur 7 ist eine Schnittansicht eines Positionierteils 84 in einer alternativen Ausführungsform dargestellt. In der gezeigten Ausführungsform ist der Bereich des Positionierteils 84 durch den das Füllmedium 30 fließt stufenförmig aufgebaut. In Richtung der zweiten Endöffnung 47 verengt sich die Stufenform des Positionierteils 84. Dieser Aufbau ermöglicht es eine Mehrzahl von zweiten Körpern 60 verschiebbar im Positionierteil 84 aufzunehmen. Dabei hat jeder zweite Körper 60, der gleichzeitig einen Ventilkörper bildet, einen eigenen Ventilsitz 42. Im vorliegenden Ausführungsbeispiel sind drei Ventile ausgebildet. Analog zur Verengung der Stufenform des Positionierteils 84 nehmen die Oberflächen 62 der zweiten Körper 60 in Richtung der zweiten Endöffnung 47 ab. Die zweiten Körper 60 sind in der gezeigten Ausführungsform kugelförmig, wobei auch eine andere Ausformung möglich ist. Weiterhin müssen die zweiten Körper 60 nicht die gleiche Form aufweisen. Ferner sind die zweiten Körper 60 über ein Kopplungselement 70 elastisch mit dem Positionierteil 84 koppelbar. Durch die Ausformung entsteht eine Durchlassöffnung 41, die der Stufenform des Positionierteils 84 folgt.

Die drei Ventile schließen durch die unterschiedlich großen umströmten Oberflächen 62 in unterschiedlichen Strömungszuständen, sodass ein Fluss des Füllmediums 30 durch die Durchlassöffnung 41 stufenweise zugelassen oder unterbunden werden kann. Hierdurch kann beispielsweise eine adaptive Sohle bereitgestellt werden, die es ermöglicht entsprechend der Anzahl an Ventilen unterschiedliche Biegesteifigkeiten aufzuweisen, sodass die Sohle dem vorherrschenden Belastungszustand besser angepasst werden kann.

Die Erfindung ist dabei nicht auf eine Ausführung mit der in Figur 7 gezeigten Anzahl an Ventilen beschränkt. Ferner können in einer alternativen, nicht gezeigten Ausführungsform in einem ersten Körper 40 eine Mehrzahl von zweiten Körpern 60 verschiebbar aufgenommen sein.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

### Bezugszeichenliste

- 1: Vorrichtung
- 20: Aufnahme
- 22: Öffnung
- 24: Innenraum
- 25: Erste Kammer
- 27: Zweite Kammer

- 30: Füllmedium

- 40: Erster Körper
- 41: Durchlassöffnung
- 42: Ventilsitz
- 43: Führungsabschnitt
- 44: Führungsoberfläche
- 45: Führung
- 46: Erste Endöffnung
- 47: Zweite Endöffnung
- 48: Endanschlag
- 49: Aussparung
- 49': Aussparung

- 50: Kraftübertragungskörper
- 52: Aussparung
- 54: Nut
- 56: Querschnittserweiterung
- 56': Querschnittserweiterung

- 60: Zweiter Körper
- 62: Oberfläche
- 64: Erster Oberflächenabschnitt
- 66: Zweiter Oberflächenabschnitt

- 70: Kopplungselement
- 72: Federsitz

- 80: Dichtabschnitt
- 82: Dichtlippe
- 84: Positionierteil

- B: Bewegungsrichtung

## Patentansprüche

1. Vorrichtung (1) zur Stabilisierung von Körpergelenken und/oder zur Unterstützung von Sportgeräten, umfassend:
eine Aufnahme (20), wobei die Aufnahme (20) mit einem Füllmedium (30) gefüllt ist,
einen ersten Körper (40) zum Interagieren mit dem Füllmedium (30), wobei der erste Körper in der Aufnahme (20) verschiebbar angeordnet ist,
einen Kraftübertragungskörper (50) zum Übertragen einer äußeren Kraft auf den ersten Körper (40),
einen zweiten Körper (60) zum Interagieren mit dem Füllmedium (30), welcher in der Aufnahme (20) verschiebbar angeordnet ist,
wobei der zweite Körper über ein Kopplungselement (70) elastisch mit dem ersten Körper (40) koppelbar ist,
**dadurch gekennzeichnet, dass**
zumindest der erste Körper (40) mindestens eine Durchlassöffnung (41) aufweist durch welche das Füllmedium (30) strömen kann,
und der zweite Körper (60) innerhalb der Durchlassöffnung (41) des ersten Körpers (40) verschiebbar aufgenommen ist, wobei der erste Körper (40) im Bereich der Durchlassöffnung (41) einen Ventilsitz (42) umfasst und der zweite Körper (60) einen Ventilkörper bildet, sodass ein Fluss des Füllmediums (30) durch die Durchlassöffnung (41) in Abhängigkeit von der Ventilstellung zugelassen oder unterbunden werden kann.

2. Vorrichtung (1) gemäß Anspruch 1, **dadurch gekennzeichnet, dass** die Durchlassöffnung (41) einen sich in einer relativen Verschieberichtung des zweiten Körpers (60) gegenüber dem ersten Körper (40) erstreckenden Führungsabschnitt (43) aufweist, um den zweiten Körper (60) in der relativen Verschieberichtung zu führen.

3. Vorrichtung (1) gemäß einem der vorhergehenden Ansprüche, **dadurch gekennzeichnet, dass** die innere Oberfläche der Durchlassöffnung (41) eine Führung (45) aufweist, um den zweiten Körper (60) in Bezug auf die innere Oberfläche der Durchlassöffnung (41) in radialer Richtung mittig zu halten.

4. Vorrichtung (1) gemäß einem der vorhergehenden Ansprüche, **dadurch gekennzeichnet, dass** der Ventilsitz (42) einen relativen Verschiebeweg des zweiten Körpers (60) gegenüber dem ersten Körper (40) in der relativen Verschieberichtung begrenzt.

5. Vorrichtung (1) gemäß einem der vorhergehenden Ansprüche, **dadurch gekennzeichnet, dass** die relativen Abmessungen des zweiten Körpers (60) gegenüber der Durchlassöffnung (41) derart ausgebildet sind, dass in der offenen Ventilstellung ein Fluidfluss des Füllmediums (30) im Bereich zwischen dem zweiten Körper (60) und der Durchlassöffnung (41) zugelassen werden kann.

6. Vorrichtung (1) gemäß einem der vorhergehenden Ansprüche, **dadurch gekennzeichnet, dass** der zweite Körper (60) in einer Ausgangsposition der Vorrichtung (1) vom Kopplungselement (70) in einer offenen Ventilposition gehalten wird.

7. Vorrichtung (1) gemäß einem der vorhergehenden Ansprüche, **dadurch gekennzeichnet, dass** das Kopplungselement (70) dazu konfiguriert ist, dass beim Einwirken von physiologischen Kräften oder Geschwindigkeiten auf die Vorrichtung (1) der zweite Körper (60) durch das Kopplungselement (70) entgegen einer Strömungskraft des Füllmediums (30) in der offenen Ventilposition gehalten werden kann, und beim Einwirken von unphysiologischen Kräften oder Geschwindigkeiten auf die Vorrichtung (1) die Strömungskraft des Füllmediums (30) den zweiten Körper (60) entgegen einer Spannkraft des Kopplungselement (70) in die geschlossene Ventilposition verschieben und darin halten kann.

8. Vorrichtung (1) gemäß einem der vorhergehenden Ansprüche, **dadurch gekennzeichnet, dass** der zweite Körper (60) derart in der Durchlassöffnung (41) des ersten Körpers (40) aufgenommen ist, dass zumindest ein Bereich der Oberfläche (62) des zweiten Körpers (60) in einer offenen Ventilstellung mit Füllmedium (30) umströmbar ist.

9. Vorrichtung (1) gemäß dem vorhergehenden Anspruch, **dadurch gekennzeichnet, dass** sich die umströmbare Oberfläche (62) des zweiten Körpers (60) von einem ersten Oberflächenabschnitt, der mindestens einer ersten Endöffnung (46) der Durchlassöffnung (41) zugewandt ist, hinzu einem zweiten Oberflächenabschnitt, der mindestens einer zweiten Endöffnung (47) der Durchlassöffnung (41) zugewandt ist, erstreckt.

10. Vorrichtung (1) gemäß einem der vorhergehenden Ansprüche, **dadurch gekennzeichnet, dass** der zweite Körper (60) zumindest teilweise ein konvexes Anströmprofil oder ein konisches Anströmprofil aufweist.

11. Vorrichtung (1) gemäß einem der Ansprüche 1 bis 9, **dadurch gekennzeichnet, dass** der zweite Körper kugelförmig ist.

12. Vorrichtung (1) gemäß einem der vorhergehenden Ansprüche, **dadurch gekennzeichnet, dass** der erste Körper (40) einen Dichtabschnitt (80), bevorzugt einen adaptiven Dichtabschnitt (80) umfasst, der zwischen dem ersten Körper (40) und der inneren Oberfläche der Aufnahme (20) angeordnet ist, um den ersten Körper (40) in der geschlossenen Ventilstellung gegenüber der Aufnahme (20) abzudichten.

13. Vorrichtung (1) gemäß Anspruch 12, **dadurch gekennzeichnet, dass** der Dichtabschnitt (80) ein Positionierteil (84) umfasst, wobei die Durchlassöffnung (41) zumindest teilweise durch das Positionierteil (84) ausgebildet ist, und wobei der zweite Körper über ein Kopplungselement (70) elastisch mit dem Positionierteil (84) koppelbar ist.

14. Vorrichtung gemäß einem der vorhergehenden Ansprüche, **dadurch gekennzeichnet, dass** der Kraftübertragungskörper (50) oder der erste Körper (40) einen Endanschlag (48) aufweist, wobei der Kraftübertragungskörper (50) von dem Endanschlag (48) in Bewegungsrichtung (B) bis zu einer Querschnittserweiterung (56) mindestens eine Aussparung (52) aufweist oder der erste Körper (40) von dem Endanschlag (48) in Bewegungsrichtung (B) bis zu einer Querschnittserweiterung (56') mindestens eine Aussparung (49') aufweist und/oder der erste Körper (40) vom Ventilsitz (42) aus entlang der Bewegungsrichtung (B) in Richtung des Kraftübertragungskörpers (50) erstreckend mindestens eine Aussparung (49) aufweist, sodass das Füllmedium durch die mindestens eine Aussparung (49,52) strömen kann.

15. Vorrichtung (1) gemäß dem vorhergehenden Anspruch, **dadurch gekennzeichnet, dass** der Dichtabschnitt (80) eine Dichtlippe (82) umfasst, wobei die Dichtlippe (82) bei geschlossener Ventilstellung mittels des Füllmediums (30) gegen die innere Oberfläche der Aufnahme (20) gedrückt werden kann und/oder der erste Körper (40) eine Kavität der Aufnahme (20) in eine erste Kammer (25) und eine zweite Kammer (27) unterteilt, wobei die Durchlassöffnung (41) in einer offenen Ventilstellung einen Fluss des Fluidmediums (30) zwischen der ersten Kammer (25) und der zweiten Kammer (27) bereitstellen kann.

## Claims

1. Apparatus (1) for stabilizing body joints and/or for supporting sports equipment, comprising:
a receptacle (20), wherein the receptacle (20) is filled with a filling medium (30),
a first body (40) for interacting with the filling medium (30), wherein the first body is arranged displaceably in the receptacle (20),
a force transmission body (50) for transmitting an external force to the first body (40),
a second body (60) for interacting with the filling medium (30), which is arranged displaceably in the receptacle (20),
wherein the second body is connectable elastically to the first body (40) via a coupling element (70),
**characterized in that**
at least the first body (40) has at least one passage opening (41) through which the filling medium (30) can flow, and
the second body (60) is received displaceably within the passage opening (41) of the first body (40), wherein the first body (40) comprises a valve seat (42) in the region of the passage opening (41) and the second body (60) forms a valve body, so that a flow of the filling medium (30) through the passage opening (41) can be permitted or prevented depending on the valve position.

2. Apparatus (1) according to claim 1, **characterized in that** the passage opening (41) has a guide section (43) extending in a relative displacement direction of the second body (60) with respect to the first body (40), in order to guide the second body (60) in the relative displacement direction.

3. Apparatus (1) according to one of the preceding claims, **characterized in that** the inner surface of the passage opening (41) has a guide (45) in order to hold the second body (60) centrally in the radial direction with respect to the inner surface of the passage opening (41).

4. Apparatus (1) according to one of the preceding claims, **characterized in that** the valve seat (42) limits a relative displacement path of the second body (60) with respect to the first body (40) in the relative displacement direction.

5. Apparatus (1) according to one of the preceding claims, **characterized in that** the relative dimensions of the second body (60) with respect to the passage opening (41) are designed such that in the open valve position a fluid flow of the filling medium (30) can be permitted in the region between the second body (60) and the passage opening (41).

6. Apparatus (1) according to one of the preceding claims, **characterized in that** the second body (60) is held in an open valve position by the coupling element (70) in a starting position of the apparatus (1).

7. Apparatus (1) according to one of the preceding claims, **characterized in that** the coupling element (70) is configured such that when physiological forces or speeds act on the apparatus (1) the second body (60) can be held in the open valve position by the coupling element (70) against a flow force of the filling medium (30), and when non-physiological forces or speeds act on the apparatus (1) the flow force of the filling medium (30) can displace the second body (60) into the closed valve position against a clamping force of the coupling element (70) and hold it therein.

8. Apparatus (1) according to one of the preceding claims, **characterized in that** the second body (60) is received in the passage opening (41) of the first body (40) such that filling medium (30) can flow around at least one region of the surface (62) of the second body (60) in an open valve position.

9. Apparatus (1) according to the preceding claim, **characterized in that** the surface (62) of the second body (60) around which flow can pass extends from a first surface section, which faces at least one first end opening (46) of the passage opening (41), to a second surface section, which faces at least one second end opening (47) of the passage opening (41).

10. Apparatus (1) according to one of the preceding claims, **characterized in that** the second body (60) at least partially has a convex incident flow profile or a conical incident flow profile.

11. Apparatus (1) according to one of Claims 1 to 9, **characterized in that** the second body is spherical.

12. Apparatus (1) according to one of the preceding claims, **characterized in that** the first body (40) comprises a sealing section (80), preferably an adaptive sealing section (80), which is arranged between the first body (40) and the inner surface of the receptacle (20) in order to seal the first body (40) with respect to the receptacle (20) in the closed valve position.

13. Apparatus (1) according to claim 12, **characterized in that** the sealing section (80) comprises a positioning part (84), wherein the passage opening (41) is formed at least partially by the positioning part (84), and wherein the second body can be elastically coupled to the positioning part (84) via a coupling element (70).

14. Apparatus according to one of the preceding claims, **characterized in that** the force transmission body (50) or the first body (40) has an end stop (48), wherein the force transmission body (50) has at least one recess (52) from the end stop (48) in the movement direction (B) up to a cross-sectional widening (56) or the first body (40) has at least one recess (49') from the end stop (48) in the movement direction (B) up to a cross-sectional widening (56') and/or the first body (40) has at least one recess (49) extending from the valve seat (42) along the movement direction (B) in the direction of the force transmission body (50), so that the filling medium can flow through the at least one recess (49, 52).

15. Apparatus (1) according to the preceding claim, **characterized in that** the sealing section (80) comprises a sealing lip (82), wherein the sealing lip (82) can be pressed against the inner surface of the receptacle (20) by means of the filling medium (30) in the closed valve position and/or the first body (40) divides a cavity of the receptacle (20) into a first chamber (25) and a second chamber (27), wherein the passage opening (41) can provide a flow of the fluid medium (30) between the first chamber (25) and the second chamber (27) in an open valve position.

## Revendications

1. Dispositif (1) de stabilisation d'articulations du corps et/ou de soutien d'appareils de sport comprenant :
un logement (20), dans lequel le logement (20) est rempli d'un fluide de remplissage (30),
un premier corps (40) pour interagir avec le fluide de remplissage (30), dans lequel le premier corps est agencé de manière déplaçable dans le logement (20),
un corps de transmission de force (50) pour la transmission d'une force extérieure au premier corps (40),
un second corps (60) pour interagir avec le fluide de remplissage (30), lequel est agencé de manière déplaçable dans le logement (20),
dans lequel le second corps peut être élastiquement couplé au premier corps actif (40) par le biais d'un élément de couplage (70),
**caractérisé en ce que**
au moins le premier corps (40) présente au moins une ouverture de passage (41) à travers laquelle le fluide de remplissage (30) peut s'écouler,
et
le second corps (60) est logé de manière déplaçable à l'intérieur de l'ouverture de passage (41) du premier corps (40), dans lequel le premier corps (40) comprend dans la zone de l'ouverture de passage (41) un siège de soupape (42), et le second corps (60) forme un corps de soupape de sorte qu'un flux du fluide de remplissage (30) à travers l'ouverture de passage (41) puisse être autorisé ou interdit en fonction de la position de soupape.

2. Dispositif (1) selon la revendication 1, **caractérisé en ce que** l'ouverture de passage (41) présente une section de guidage (43) s'étendant dans un sens de déplacement relatif du second corps (60) par rapport au premier corps (40) afin de guider le second corps (60) dans le sens de déplacement relatif.

3. Dispositif (1) selon l'une quelconque des revendications précédentes, **caractérisé en ce que** la surface intérieure de l'ouverture de passage (41) présente un guidage (45) afin de retenir le second corps (60) au milieu dans le sens radial par rapport à la surface intérieure de l'ouverture de passage (41).

4. Dispositif (1) selon l'une quelconque des revendications précédentes, **caractérisé en ce que** le siège de soupape (42) délimite une course de déplacement relative du second corps (60) par rapport au premier corps (40) dans le sens de déplacement relatif.

5. Dispositif (1) selon l'une quelconque des revendications précédentes, **caractérisé en ce que** les dimensions relatives du second corps (60) par rapport à l'ouverture de passage (41) sont configurées de telle manière que dans la position de soupape ouverte, un flux fluidique du fluide de remplissage (30) puisse être autorisé dans la zone entre le second corps (60) et l'ouverture de passage (41).

6. Dispositif (1) selon l'une quelconque des revendications précédentes, **caractérisé en ce que** le second corps (60) est maintenu dans une position de départ du dispositif (1) par l'élément de couplage (70) dans une position de soupape ouverte.

7. Dispositif (1) selon l'une quelconque des revendications précédentes, **caractérisé en ce que** l'élément de couplage (70) est configuré afin que lors de l'action de forces ou de vitesses physiologiques sur le dispositif (1), le second corps (60) puisse être maintenu par l'élément de couplage (70) à l'encontre d'une force d'écoulement du fluide de remplissage (30) dans la position de soupape ouverte, et lors de l'action de forces ou de vitesses non physiologiques sur le dispositif (1), la force d'écoulement du fluide de remplissage (30) puisse déplacer le second corps (60) à l'encontre d'une force de serrage de l'élément de couplage (70) dans la position de soupape fermée et l'y maintenir.

8. Dispositif (1) selon l'une quelconque des revendications précédentes, **caractérisé en ce que** le second corps (60) est logé dans l'ouverture de passage (41) du premier corps (40) de telle manière qu'au moins une zone de la surface (62) du second corps (60) puisse être contournée par du fluide de remplissage (30) dans une position de soupape ouverte.

9. Dispositif (1) selon la revendication précédente, **caractérisé en ce que** la surface (62) contournable du second corps (60) s'étend d'une première section de surface qui est tournée vers au moins une première ouverture d'extrémité (46) de l'ouverture de passage (41), jusqu'à une seconde section de surface qui est tournée vers au moins une seconde ouverture d'extrémité (47) de l'ouverture de passage (41).

10. Dispositif (1) selon l'une quelconque des revendications précédentes, **caractérisé en ce que** le second corps (60) présente au moins partiellement un profilé d'afflux convexe ou un profilé d'afflux conique.

11. Dispositif (1) selon l'une quelconque des revendications 1 à 9, **caractérisé en ce que** le second corps est sphérique.

12. Dispositif (1) selon l'une quelconque des revendications précédentes, **caractérisé en ce que** le premier corps (40) comprend une section étanche (80), de préférence une section étanche (80) adaptative qui est agencée entre le premier corps (40) et la surface intérieure du logement (20) afin de rendre étanche le premier corps (40) par rapport au logement (20) dans la position de soupape fermée.

13. Dispositif (1) selon la revendication 12, **caractérisé en ce que** la section étanche (80) comprend une partie de positionnement (84), dans lequel l'ouverture de passage (41) est configurée au moins partiellement par la partie de positionnement (84), et dans lequel le second corps peut être couplé élastiquement à la partie de positionnement (84) par le biais d'un élément de couplage (70).

14. Dispositif selon l'une quelconque des revendications précédentes, **caractérisé en ce que** le corps de transmission de force (50) ou le premier corps (40) présente une butée d'extrémité (48), dans lequel le corps de transmission de force (50) présente au moins un évidement (52) depuis la butée d'extrémité (48) dans le sens de déplacement (B) jusqu'à un élargissement de section (56) ou le premier corps (40) présente au moins un évidement (49') depuis la butée d'extrémité (48) dans le sens de déplacement (B) jusqu'à un élargissement de section (56') et/ou le premier corps (40) présente au moins un évidement (49) s'étendant depuis le siège de soupape (42) le long du sens de déplacement (B) en direction du corps de transmission de force (50), de sorte que le fluide de remplissage puisse s'écouler à travers le au moins un évidement (49, 52).

15. Dispositif (1) selon la revendication précédente, **caractérisé en ce que** la section étanche (80) comprend une lèvre d'étanchéité (82), dans lequel la lèvre d'étanchéité (82) peut être pressée au moyen du fluide de remplissage (30) contre la surface intérieure du logement (20) en cas de position de soupape fermée et/ou le premier corps (40) divise une cavité du logement (20) en une première chambre (25) et une seconde chambre (27), dans lequel l'ouverture de passage (41) peut fournir dans une position de soupape ouverte un flux du fluide de remplissage (30) entre la première chambre (25) et la seconde chambre (27).
